# DEMANDE DE BREVET EUROPEEN

(11) **EP 4 382 615 A1**
(43) Date de publication de la demande: **12.06.2024**
(21) Numéro de dépôt: 23211379.5
(22) Date de dépôt: 22.11.2023
(51) Int. Cl.: C12Q 1/6806

(54) **PROCÉDÉ DE DÉTECTION D'UNE ESPÈCE ALLERGÈNE, CONTAMINANTE DE NATURE FRAUDULEUSE OU DE NATURE OGM DANS UN ÉCHANTILLON AGRO-ALIMENTAIRE**

(30) Priorité: 07.12.2022 FR 2212915
(71) Demandeur: Commissariat à l'énergie atomique et aux énergies alternatives, 75015 Paris (FR)
(72) Inventeur: BOURDAT, Anne-Gaëlle, 38054 Grenoble Cedex 09 (FR); CUBIZOLLES, Myriam-Laure, 38054 Grenoble Cedex 09 (FR); DEN DULK, Remco, 38054 Grenoble Cedex 09 (FR); CLAREBOUT, Gervais, 38054 Grenoble Cedex 09 (FR); SERRANO, Bastien, 38054 Grenoble Cedex 09 (FR); LAURENT, Patricia, 38054 Grenoble Cedex 09 (FR)
(74) Mandataire: INNOV-GROUP

(57) **Abrégé**

L'invention concerne un procédé de détection d'une espèce cible, de type allergène, contaminante ou de nature frauduleuse dans un échantillon (ECH) pouvant comporter des inhibiteurs, le procédé consistant à :
- Obtenir un précipité (P_2) contenant des molécules d'ADN de l'espèces cible ;
- Filtrer le filtrat contenant ledit deuxième précipité (P_2) contre un deuxième filtre (FT_2), en vue de séparer des deuxièmes composés (C_2) issus dudit deuxième précipité (P_2),
- Eluer des molécules d'ADN de l'espèce cible présente dans les deuxièmes composés issus du deuxième précipité (P_2) à travers le deuxième filtre (FT_2), par ajout d'un tampon d'élution (TE_1) choisi pour dissoudre lesdites molécules d'ADN, et de récupération d'un éluat (E_1) ;
- Détecter des molécules d'ADN de l'espèce cible présentes dans ledit éluat (E_1) ;

## Description

### Domaine technique de l'invention

La présente invention se rapporte à un procédé de détection d'une espèce allergène, contaminante, de nature frauduleuse ou de nature OGM dans un échantillon agro-alimentaire.

### Etat de la technique

La présence d'espèces allergènes, contaminantes ou de nature frauduleuse dans un échantillon agro-alimentaire est un sujet sensible.

A titre d'exemple, les groupes d'aliments allergènes sont :
- Les céréales contenant du gluten (blé, seigle, orge, avoine, épeautre, kamut ou leurs souches hybridées) et produits à base de ces céréales ;
- Les crustacés et produits à base de crustacés ;
- Œufs et produits à base d'oeufs ;
- Poissons et produits à base de poissons ;
- Arachides et produits à base d'arachide ;
- Soja et produits à base de soja ;
- Lait et produits à base de lait (y compris de lactose) ;
- Fruits à coques (amandes, noisettes, noix, noix de : cajou, pécan, macadamia, du Brésil, du Queensland, pistaches) et produits à base de ces fruits ;
- Céleri et produits à base de céleri ;
- Moutarde et produits à base de moutarde ;
- Graines de sésame et produits à base de graines de sésame ;
- Anhydride sulfureux et sulfites en concentration de plus de 10mg/kg ou 10 mg/l (exprimés en SO2).
- Lupin et produits à base de lupin ;
- Mollusques et produits à base de mollusques ;

A titre d'exemple, les espèces contaminantes peuvent aussi être des protéines, les mycotoxines, les bactéries, les virus, les cellules, les pesticides, les antibiotiques, les perturbateurs endocriniens.

A titre d'exemple, les espèces de nature frauduleuse sont des composés supposés non présents dans l'aliment comme la farine ou le sucre dans la poudre d'amande, de la viande de cheval mélangée avec de la viande de boeuf, des épices de curry dans du safran.

Une espèce allergène peut avoir de graves effets à très faibles quantités, dès les seuils de quelques ppm, ce qui représente quelques milligrammes d'allergènes par kilo d'aliment. A titre d'exemple concernant le gluten, la norme 118-1979 du Codex Alimentarius, la concentration en gluten dans les aliments ne doit pas dépasser 20 mg/kg (20 ppm) pour les produits étiquetés "sans gluten".

Le secteur agro-agro-alimentaire a aujourd'hui besoin de disposer de tests fiables, sensibles, robustes, automatisés, rapides, faciles à mettre en oeuvre et réalisables directement sur les sites de production. De même, pour leur permettre de détecter la présence d'espèces allergènes ou contaminantes dans leurs aliments, les utilisateurs devraient pouvoir disposer de dispositifs performants mettant en oeuvre un test fiable et rapide.

Les espèces allergènes ou contaminantes agro-alimentaires :
- Sont pour l'essentiel des protéines ou des petites molécules parfois dégradées ou au contraire, dont le pouvoir allergène est révélé ou augmenté par les procédés de fabrication/transformation ;
- Sont insérées dans des matrices complexes ;
- Evoluent au cours de la durée de vie du produit et de son éventuelle transformation (cuisson, congélation,...) ;
- Peuvent être introduites fortuitement ou non
- Sont allergisantes, même dans des très faibles quantités, de l'ordre de quelques ppm ;

Pour rappel, schématiquement, une molécule d'ADN va être transcrite en ARN qui sera ensuite traduit en protéine. En théorie, le même type d'information se trouve à la fois sur l'ADN initial et sur la protéine. Cependant, pour différentes raisons, ce n'est pas toujours le cas :
- Il existe une multitude de transcrits possibles à partir d'un même ADN ;
- Il existe une multitude de traductions possible en protéine à partir d'un ARN ;
- Les processus de fabrication (chauffage, rôtissage, congélation, exposition, mélange à d'autres substances...) sont susceptibles de modifier les protéines, ces processus pouvant supprimer un pouvoir allergène ou au contraire révéler et rendre accessible une zone allergène de la protéine ;

La mise en oeuvre d'une méthode de détection fiable et rapide peut donc s'avérer plus complexe qu'on ne pourrait le penser. De récentes études (publication référencée : Anne C. Eischeid, Sarah R. Stadig & Prasad Rallabhandi (2021) Comparison of real-time PCR and ELISA for the détection of crustacean shellfish allergens, Food Additives & Contaminants: Part A, 38:4, 563-572, DOI: 10.1080/19440049.2021.1874061) soulignent d'ailleurs l'importance de l'utilisation de différentes méthodes orthogonales pour garantir la robustesse des analyses.

Il existe plusieurs méthodes d'extraction et de purification de l'ADN. Elles peuvent être classées en trois catégories :
- La première méthode qui comporte des étapes de lyse, dénaturation et purification par précipitation de l'ADN ;
- La deuxième méthode qui comporte des étapes de lyse, dénaturation et purification par affinité de l'ADN avec un support ;
- La troisième méthode qui comporte des étapes de lyse, dénaturation et purification de l'ADN par changement de phase ;

Beaucoup de procédés et dispositifs ont déjà été proposés dans l'état de la technique. C'est le cas dans les demandes de brevets WO2017/059103A1**,** WO2008/110655A1**,** US2016/266083A1 et dans le brevet US7585529B2**.**

Certains de ces documents antérieurs proposent des solutions de détection d'espèces allergènes par analyse biomoléculaire, d'autres des solutions de détection d'espèces allergènes par analyse immunologique.

Les méthodes de détection par analyse biomoléculaire présentent globalement les inconvénients suivants :
- Elles sont uniquement dédiées à l'analyse biomoléculaire ;
- Elles peuvent être toxiques ;
- Elles doivent être mises en oeuvre à température plus élevée que la température ambiante ;
- Elles nécessitent une centrifugeuse et un appareillage spécifique de thermocyclage, chauffage et détection de fluorescence, ce qui les rendent difficiles à mettre en oeuvre sur le terrain ;
- Elles nécessitent une étape de broyage de l'échantillon prélevé, entraînant une perte potentielle de sensibilité à la détection, par dilution des traces recherchées dans un large broyat ;

Les méthodes de détection par analyse immunologique présentent souvent les inconvénients suivants :
- Elles sont uniquement dédiées à l'analyse immunologique ;
- Elles peuvent être toxiques, notamment du fait de l'utilisation classique du 2-mercaptoéthanol connu pour ses effets CMR (cancérogène, mutagène et toxique pour la reproduction) ;
- Elles peuvent être longues ;
- Elles peuvent nécessiter des manipulations sous hotte et l'emploi d'une centrifugeuse ;
- Elles peuvent occasionner une inactivation de l'anticorps ajouté pour la détection ;

La demande de brevet US2010/273254A1 décrit un procédé de purification d'ADN plasmidique et d'élimination de l'ADN « hôte » qui comporte les étapes suivantes :
- Lyse des cellules ;
- Filtration pour retirer les débris de cellules et les impuretés, dont une partie de l'ADN chromosomique de la cellule hôte ;
- Précipitation en utilisant le polyéthylène glycol afin de précipiter l'ADN plasmidique ;
- Collecte de l'ADN plasmidique partiellement purifié par filtration du précipité et élimination d'une partie de l'ADN chomosomique de la cellule hôte;
- Précipitation des impuretés (polysaccharides et une partie de l'ADN chomosomique de la cellule hôte) à l'aide d'Acétate d'ammonium ;
- Filtration des impuretés ;
- Séparation de l'ADN plasmidique par chromatographie ;

Ce document antérieur concerne la haute purification d'ADN plasmidique pour un usage thérapeutique et le procédé décrit n'est pas applicable à la détection d'une espèce cible telle qu'une espèce allergène, contaminante, de nature frauduleuse ou de nature OGM. La technique est destinée à la production de plasmide à large échelle, généralement dans des bactéries, en vue de l'injection à des patients dans un but thérapeutique. L'élimination de l'ADN hôte chromosomique bactérien et des autres impuretés bactériennes est essentiel.

Le but de l'invention est de proposer un procédé de détection d'une espèce allergène, contaminante, de nature frauduleuse ou de nature OGM dans un échantillon agro-alimentaire, pouvant être une matière première brute ou transformée, ou sous forme d'assemblage, et pouvant être complexe (éventuellement végétale, avec du gras, des protéines) qui soit :
- Fiable,
- Robuste,
- Sensible,
- Eventuellement le plus automatisé possible, de manière à être simple et facile à mettre en oeuvre.

### Exposé de l'invention

Ce but est atteint par un procédé de détection d'une espèce cible, de type allergène, contaminante, de nature frauduleuse ou de nature OGM dans un échantillon pouvant comporter des inhibiteurs, ledit procédé comportant :
- Une étape d'attaque chimique par mélange dudit échantillon avec une solution corrosive en vue d'obtenir un extrait en solution ;
- Une étape d'ajout d'un premier tampon de précipitation audit extrait en solution obtenu pour obtenir un premier précipité d'au moins une partie des inhibiteurs de réactions contenus dans ledit extrait en solution ;
- Une étape de filtration dudit extrait en solution contre un premier filtre, en vue d'éliminer des premiers composés autres que les acides nucléiques, issus dudit premier précipité, et de récupération d'un filtrat après filtration, déchargé desdits premiers composés ;
- Une étape d'ajout d'un deuxième tampon de précipitation audit filtrat en vue d'obtenir un deuxième précipité contenant des molécules d'ADN cellulaire de l'espèces cible ;
- Une étape de filtration dudit filtrat contenant ledit deuxième précipité contre un deuxième filtre, en vue de séparer des deuxièmes composés issus dudit deuxième précipité, ledit deuxième filtre étant choisi pour ne pas laisser passer lesdites molécules d'ADN cellulaire précipitées de l'espèce cible ;
- Une étape d'élution des molécules d'ADN cellulaire de l'espèce cible présente dans les deuxièmes composés issus du deuxième précipité à travers le deuxième filtre, par ajout d'un tampon d'élution choisi pour dissoudre lesdites molécules d'ADN, et de récupération d'un éluat ;
- Une étape de détection des molécules d'ADN cellulaire de l'espèce cible présentes dans ledit éluat ;

Selon une particularité, le procédé comporte également une étape de lavage des deuxièmes composés issus du deuxième précipité par passage d'un tampon de lavage à travers le deuxième filtre.

Selon une autre particularité, le tampon de lavage est à base d'alcool ou d'alcool et d'eau, ou d'alcool, eau et sels.

Selon une autre particularité, le procédé comporte une étape de séchage des deuxièmes composés obtenus après filtration sur le deuxième filtre.

Selon une autre particularité, la solution corrosive comporte un ou plusieurs des composants de la liste suivante :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS, Tergitol),
- Chlorure de Sodium ou de Potassium.

Selon une autre particularité, le premier tampon de précipitation comporte un ou plusieurs des composants de la liste suivante :
- Acétate d'ammonium,
- Polyvinylpyrrolidone.

Selon une autre particularité, le deuxième tampon de précipitation est un alcool.

Selon une autre particularité, l'alcool utilisé comme deuxième tampon de précipitation est l'éthanol ou l'isopropanol.

Selon une autre particularité, le tampon d'élution est choisi parmi l'eau et au moins un réactif utilisé pour la mise en oeuvre d'une réaction d'amplification.

Selon une autre particularité, l'éluat est injecté dans plusieurs chambres fluidiques en parallèle.

Selon une autre particularité, l'étape d'ajout du deuxième tampon de précipitation et les étapes suivantes sont mises en oeuvre dans une carte microfluidique.

### Brève description des figures

D'autres caractéristiques et avantages vont apparaître dans la description détaillée qui suit faite en regard des dessins annexés dans lesquels :
- La figure 1 illustre les différentes étapes mises en oeuvre dans l'invention ;
- La figure 2 illustre le principe de l'attaque chimique réalisée sur l'échantillon agro-alimentaire prélevé ;
- La figure 3 montre un exemple d'une carte micro-fluidique pouvant être employée pour la mise en oeuvre du procédé de l'invention ;

### Description détaillée d'au moins un mode de réalisation

Le procédé de détection d'une espèce allergène, d'une espèce contaminante, de nature frauduleuse ou de nature OGM est mis en oeuvre sur un échantillon, avantageusement un échantillon agro-alimentaire ECH. Dans la suite de la description, de manière non limitative, nous ferons référence à un échantillon agro-alimentaire ECH.

Dans la suite de la description, nous ferons référence à l'espèce cible pour évoquer l'espèce allergène, l'espèce contaminante ou l'espèce de nature frauduleuse recherchée.

L'invention s'applique à la détection de l'espace cible à partir de son ADN cellulaire (appelé simplement ADN dans la description ci-dessous). Cet ADN cellulaire est distinct de l'ADN plasmidique également connu et formé de molécules d'ADN circulaires. Les plasmides sont plus souvent à usage thérapeutique et employés pour préparer des anticorps, réaliser des analyses génétiques mais leur isolation/purification dans le cadre de l'invention ne présente pas d'intérêt particulier.

L'échantillon agro-alimentaire ECH peut être de tous types, par exemple une matière première comme des graines, ou un produit transformé comme de la farine, une pâte (par exemple pâte à tartiner) ou être formé de tout prélèvement d'une matrice agro-alimentaire réalisée sur une chaîne de fabrication ou directement par le consommateur final lors d'un repas (ex : produit fini tel que sauce bolognaise, cassoulet, etc...). L'échantillon peut également être constitué d'une lingette ou d'un écouvillon récupéré à l'issue d'un prélèvement de surface sur une chaine de production pour vérifier l"efficacité d'un nettoyage, ou d'une eau de rinçage après un nettoyage.

On verra que le procédé de l'invention présente un intérêt particulier lorsque la matrice agro-alimentaire est hétérogène (graines, morceaux...).

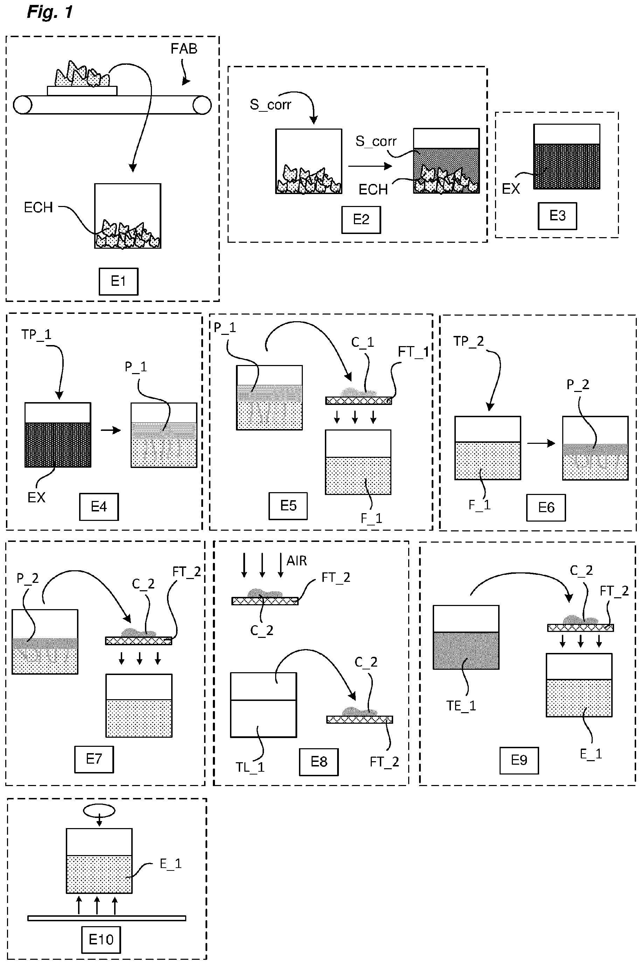

En référence à la figure 1, après une étape de prélèvement E1 de l'échantillon agro-alimentaire ECH (par exemple sur une chaîne de fabrication FAB), le procédé comporte ainsi une étape de préparation E2 visant à réaliser une attaque chimique de l'échantillon agro-alimentaire ECH prélevé, par mélange dudit échantillon agro-alimentaire avec une solution corrosive S_corr. Suite à cette attaque chimique, on obtient ensuite un extrait EX en solution.

A titre d'exemple, la solution corrosive S_corr peut comporter des agents chaotropiques, des agents acides, des agents basiques, des solutions oxydantes, des solutions détergentes, des sels.

La solution corrosive peut comporter un ou plusieurs des composants suivants :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS, Tergitol),
- Chlorure de Sodium ou de Potassium.

Avantageusement, la solution corrosive choisie doit permettre la précipitation ultérieure des acides nucléiques dans l'alcool choisi (par exemple l'éthanol). Pour être réalisée efficacement à température ambiante, celle-ci doit comporter des sels de type Chlorure de Sodium ou de Potassium.

Dans un autre mode de réalisation, ces sels pourraient cependant être ajoutés ultérieurement au moment de l'étape E6 décrite ci-dessous, correspondant à une précipitation des molécules d'ADN par ajout d'un tampon de précipitation TP_2 (voir ci-après).

Selon une particularité, l'échantillon agro-alimentaire ECH est mélangé avec la solution corrosive S_corr dans un ratio d'un volume d'échantillon sur un volume de solution corrosive qui est compris entre 0.1 et 1000, préférentiellement compris entre 1 et 100 et préférentiellement compris entre 1 et 10, idéalement égal à 4.

Idéalement un volume d'échantillon est donc dilué dans quatre volumes de solution corrosive.

Dans certains cas, les quantités à détecter sont très faibles dans l'échantillon agro-alimentaire ECH prélevé et peuvent être présentes simplement à l'état de traces. Le fait de réaliser une attaque chimique sur l'échantillon agro-alimentaire permet de récupérer les poussières et de surreprésenter les traces de l'espèce cible présente dans l'échantillon agro-alimentaire.

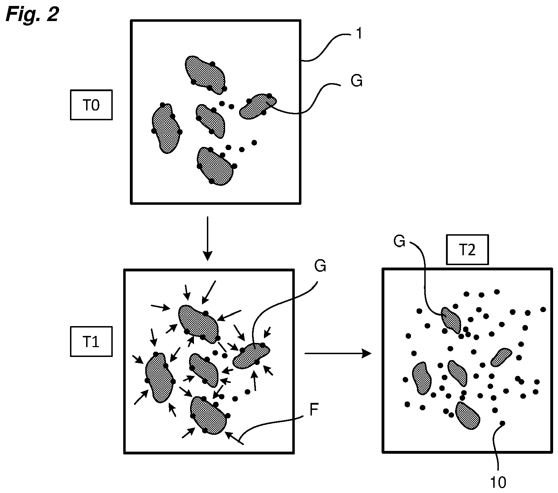

Ceci est particulièrement efficace lorsque l'échantillon agro-alimentaire ECH est de type hétérogène, par exemple composé de graines ou de morceaux. Dans ce cas particulier, l'attaque chimique va permettre d'attaquer les graines ou morceaux en surface pour détacher les traces de l'espèce cible et ainsi les rendre plus facilement détectables. Ce principe est notamment illustré par la figure 2.

Sur cette figure 2 :
A T0 : les graines G sont prélevées dans un réceptacle 1 ;
A T1 : La solution corrosive est ajoutée. Les flèches F illustrent le principe d'attaque chimique sur les graines G.
A T2 : Grâce à l'attaque chimique, les traces 10, poussières ainsi que l'enveloppe de la graine sont détachées de la surface des graines G, permettant une surreprésentation de la cible recherchée.

Selon un aspect particulier de l'invention, lorsque l'échantillon agro-alimentaire ECH prélevé est dans un état hétérogène, il n'est donc nul besoin de le broyer avant l'étape de préparation E2 par attaque chimique, ce qui permet de gagner du temps sur l'ensemble du process d'analyse tout en améliorant l'efficacité de la détection, ainsi que de diminuer les coûts et les risques potentiels de contamination.

Après l'étape de préparation E2, on obtient un extrait en solution (EX - E3). On prélève une fraction de l'extrait en solution EX (1/40).

L'étape suivante E4 consiste à ajouter un tampon de précipitation TP_1 dans le prélèvement de l'extrait en solution EX obtenu.

On ajoute par exemple un volume du tampon de précipitation TP_1 pour deux volumes de l'extrait en solution EX.

Il s'agit alors de précipiter certains composés (autres que les acides nucléiques) de l'échantillon agro-alimentaire, notamment des protéines, polyphénols, polysaccharides, lipides, gras si l'échantillon agro-alimentaire en contient.

Le tampon de précipitation TP_1 est par exemple choisi parmi un ou plusieurs des composants tels que l'acétate d'ammonium 7.5M et l'eau. Par ailleurs, l'ajout d'un composé tel que le Polyvinylpyrrolidone 30 (appelé également PVP30) à 3% est utile lorsque l'échantillon agro-alimentaire comporte une matrice de type végétal.

On obtient ainsi un premier précipité P_1.

L'étape suivante E5 consiste à filtrer lesdits composés C_1 issus du précipité P_1 obtenu lors de l'étape précédente. Pour cela, l'extrait en solution contenant le précipité P_1 est passé à travers un filtre FT_1.

Ce filtre FT_1 peut être par exemple de type seringue à gradient.

Par cette étape de filtration, on élimine les composés C_1 du précipité P_1 et on obtient en aval du filtre un filtrat F_1 contenant les acides nucléiques en solution.

L'étape suivante E6 consiste à ajouter un deuxième tampon de précipitation TP_2 dans le filtrat F_1 obtenu lors de l'étape précédente E5.

Il s'agit alors d'obtenir un nouveau précipité P_2 contenant les molécules d'ADN de l'espèce cible.

Ce tampon de précipitation TP_2 est par exemple un alcool, tel que par exemple l'éthanol. Idéalement on utilise de l'éthanol à 96%. Pour rappel, il peut s'avérer utile d'ajouter des sels lors de cette étape E6, si ceux-ci n'avaient pas été ajoutées initialement à l'étape E2.

L'étape suivante E7 consiste à filtrer le filtrat F_1 contenant le précipité P_2 obtenu lors de l'étape E6 sur un deuxième filtre FT_2, en vue de séparer les composés C_2 du précipité P_2 contenant les molécules d'ADN de l'espèce cible. Le filtre FT_2 laisse passer la partie liquide du filtrat et retient la partie solide issue C_2 du précipité P_2 et contenant les molécules d'ADN de l'espèce cible.

Lors de cette étape de filtration, le filtre FT_2 utilisé est choisi avec une taille de pores adaptée pour retenir la partie solide C_2 du précipité P_2.

Les composés solides retenus par le filtre FT_2 sont les composés C_2 d'intérêt contenant les molécules d'ADN de l'espèce cible. La partie liquide ayant traversé le filtre FT_2 est éliminée.

L'étape suivante E8 peut consister à venir laver les composés C_2 solides retenus par le filtre par passage d'un tampon de lavage TL_1 à travers le filtre puis à les sécher.

Le séchage peut consister à passer un flux d'air (AIR) à travers le filtre FT_2.

Le tampon de lavage TL_1 utilisé peut être un alcool tel que par exemple l'éthanol 70%. Le séchage permet d'éliminer l'alcool qui est un inhibiteur de PCR.

L'étape suivante E9 consiste à récupérer les molécules d'ADN de l'espèce cible, retenues par le filtre FT_2. Il s'agit alors d'ajouter un tampon d'élution TE_1 sur les composés C_2 retenus sur le filtre FT_2 pour dissoudre les molécules d'ADN de l'espèce cible à travers le filtre. On obtient ensuite un éluat E_1 sous forme entièrement liquide dans lequel les molécules d'ADN de l'espèce cible ont été dissoutes.

Le tampon d'élution TE_1 peut être l'eau ou directement formé d'un réactif d'amplification.

Le réactif d'amplification peut être par exemple :
- De type qPCR 1X (ici le luna qPCR de NEB en 1X dans lequel ont été rajouté des amorces et sondes qPCR spécifiques de la cible recherchée dans les concentrations recommandées par le fournisseur de réactif), ou
- De type qPCR 2X (il faudra ajouter en liquide à posteriori les amorces et sondes qPCR spécifiques de la cible recherchée dans les concentrations recommandées par le fournisseur de réactif), ou
- De type qPCR 1X (il faudra ajouter à posteriori les amorces et sondes qPCR spécifiques de la cible recherchée dans les concentrations recommandées par le fournisseur de réactif, celles-ci pouvant être à l'état séché et/ou lyophilisé dans le consommable de réaction utilisé pour la réaction qPCR).

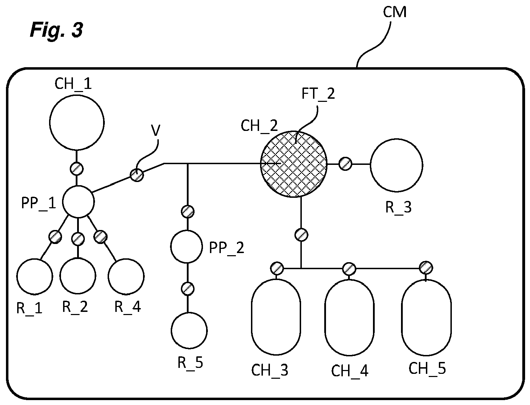

L'étape suivante E10 consiste à venir détecter qualitativement et quantitativement les molécules d'ADN de l'espèce cible dans l'éluat E_1 obtenu après l'élution de l'étape E8. La réaction mise en oeuvre peut être une réaction d'amplification biomoléculaire. L'éluat E_1 obtenu lors de l'étape E10 est par exemple réparti dans plusieurs chambres d'amplification distinctes (voir description en liaison avec la figure 3). Si le tampon d'élution TE_1 utilisé lors de l'étape précédente est le réactif d'amplification, il s'agit de venir placer dans chaque chambre d'amplification les amorces et sondes spécifiques de la cible recherchée. Comme décrit ci-dessus, les amorces et sondes peuvent être préalablement placées dans la chambre sous une forme séchée et/ou lyophilisée.

La réaction d'amplification mise en oeuvre dans chaque chambre est par exemple de type qPCR.

Selon un aspect particulier de l'invention, le procédé de l'invention décrit ci-dessus peut être automatisé au moins en partie.

Cette automatisation est permise par l'emploi d'une carte microfluidique CM dans laquelle est créé un réseau fluidique, et de divers moyens d'actionnement permettant de commander des déplacements de fluide dans ledit réseau fluidique.

Des vannes V sont prévues sur les canaux fluidiques du réseau, ainsi que des pompes pour aspirer ou pousser les fluides dans le réseau. Les moyens d'actionnement, par exemple pneumatiques, permettent d'actionner chaque vanne et pompe de manière indépendante.

La figure 3 montre un exemple d'une telle carte microfluidique CM. Celle-ci est montrée à titre d'exemple de réalisation et permet d'illustrer simplement l'architecture d'une telle carte.

Les étapes E6 à E10 peuvent notamment être mises en oeuvre dans la carte microfluidique.

Lors de l'étape E6, le filtrat F_1 et le tampon de précipitation TP_2 sont aspirés dans deux réservoirs R_1, R_2 distincts de la carte microfluidique.

Ainsi le filtrat F_1 peut être aspiré du réservoir R_1 et poussé vers la chambre de mélange CH_1, par actionnement de la pompe PP_1. Ensuite, le tampon de précipitation TP_2 peut être aspiré de R_2 et poussé vers la chambre de mélange CH_1 également. Ensuite, des allers-retours entre la pompe PP_1 et la chambre CH_1 peuvent être effectués pour améliorer le mélange.

L'étape E7 est mise en oeuvre en envoyant le filtrat F_1 contenant le précipité P_2 obtenu vers une chambre CH_2, séparée en deux espaces par le filtre FT_2. Le filtre FT_2 retient les molécules d'ADN précipité de l'espèce cible. Le reste du filtrat F_1 ayant traversé le filtre FT_2 est envoyé vers un réservoir poubelle R_3.

L'étape E8 de lavage peut être réalisée, à l'aide la pompe PP_1, en injectant le tampon de lavage TL_1 présent dans un autre réservoir R_4 de la carte microfluidique à travers les composés retenus sur le filtre FT_2. Un séchage peut être réalisé en envoyant un flux d'air à travers le filtre FT_2. Pour ce lavage, un passage par la chambre de mélange CH_2 est avantageux afin de la rincer et d'assurer ainsi que la totalité du contenu en ADN précipité soit emportée vers le filtre FT_2.

L'étape E9 peut être réalisée en injectant le tampon d'élution TE_1 présent dans un autre réservoir R_5 de la carte contre les composés présents dans CH_2 et retenus sur le filtre FT_2, en vue de les dissoudre et de les faire passer à travers le filtre FT_2. Cette opération est réalisée à l'aide d'une autre pompe PP_2 afin d'éviter de contaminer le tampon d'élution avec les autres liquides (notamment ceux contenus dans R1 et R2), ce qui pourrait perturber le fonctionnement de l'amplification ADN.

L'éluat E_1 obtenu à l'issue de l'étape E9 peut être injecté dans plusieurs chambres d'amplification CH_3, CH_4, CH_5 agencées en parallèle dans la carte microfluidique.

L'étape E10 relative à la détection de l'ADN des espèces cibles par amplification biomoléculaire peut être réalisée directement dans chaque chambre de la carte microfluidique.

Une réalisation concrète est détaillée ci-dessous :
Etapes E1+E2 + E3 : On prélève 10g d'échantillon. Il est possible de doper l'échantillon de manière artificielle, par exemple en ajoutant quelques ppm de contaminant, un fragment d'amande ou un grain de blé.

On ajoute 40mL de la solution corrosive choisie (KOH 0,9M, TERGITOL 15-S-9 5% p/v, Tween 80 5% p/v, NaCl 2M, H₂O).

On agite puis on laisse incuber pendant 10 minutes.

On rajoute 40mL d'eau.

Etapes E4+E5 : On ajoute à 2mL de l'extrait obtenu précédemment 1mL d'acétate d'ammonium 7.5M et 3% de PVP30 (à température ambiante.

On filtre le mélange obtenu sur le filtre FT_1 à gradient.

On récupère le filtrat F_1.

Etapes E6+E7+E8 : On mélange 125µL du filtrat F_1 et 125µL d'éthanol 96%.

On laisse incuber 2 minutes.

On filtre par aspiration le filtrat F_1 incluant le précipité P_2 à travers le filtre FT_2.

On lave avec 250µL d'éthanol 70% par aspiration jusqu'à sécher les composés C_2.

On lave à nouveau avec 250µL d'éthanol 70% par aspiration jusqu'à sécher les composés C_2.

On sèche à l'air par aspiration ou pression.

Etape E9 : On élue l'ADN avec 100 µL d'eau ou d'un réactif PCR (mix LUNA) par aspiration et refoulement (par exemple 2 allers-retours).

En résultats, on obtient après une réaction de type qPCR :

| | Masse | Kit Gluten |
|---|---|---|
| | | Ct obtenu en qPCR |
| 1 grain de blé | ~40mg | 27 |
| Grain retiré | traces | 31 |

En qPCR, une détection positive (mesure Ct) indique la présence de gluten. Plus la valeur de Ct est faible, plus la quantité de gluten est forte. Dans cet exemple, un échantillon contenant un grain de blé est détecté positif au gluten. L'échantillon dans lequel un grain de blé a été déposé, puis retiré, est également détecté positif. Ceci indique que les poussières "laissées" dans l'échantillon ont contaminé l'échantillon en gluten.

On comprend de ce qui précède que l'invention présente ainsi de nombreux avantages, parmi lesquels :
- Une simplicité de mise en oeuvre, grâce à l'utilisation de moyens classiques et la possibilité d'une automatisation au moins partielle dans une carte microfluidique ;
- Une diminution du risque de contamination de l'environnement. L'échantillon est mis en solution dès le début du protocole pour limiter le risque de dispersion des poussières dans l'environnement. Ainsi, l'absence de broyage de l'échantillon limite aussi le risque de contamination.
- Un niveau de robustesse élevé ;
- La possibilité de conserver toutes les espèces cibles du début à la fin du procédé, sans fragmentation ;
- Une solution fiable car mettant en jeu des manipulations simples et éprouvées et qui n'utilisent avantageusement pas la centrifugation (solution essentiellement à base de précipitation et de filtration) ;
- Une solution adaptable à des échantillons complexes, pouvant comporter des morceaux, une matrice végétale ;

## Revendications

1. Procédé de détection d'une espèce cible, de type allergène, contaminante, de nature frauduleuse ou de nature OGM, dans un échantillon (ECH) pouvant comporter des inhibiteurs, **caractérisé en ce qu'**il comporte :
- Une étape (E2) d'attaque chimique par mélange dudit échantillon avec une solution corrosive (S_corr) en vue d'obtenir un extrait en solution (EX) ;
- Une étape (E4) d'ajout d'un premier tampon de précipitation (TP_1) audit extrait en solution obtenu pour obtenir un premier précipité (P_1) d'au moins une partie des inhibiteurs de réactions contenus dans ledit extrait en solution (EX) ;
- Une étape (E5) de filtration dudit extrait en solution contre un premier filtre (FT_1), en vue d'éliminer des premiers composés (C_1) autres que les acides nucléiques, issus dudit premier précipité (P_1), et de récupération d'un filtrat (F_1) après filtration, déchargé desdits premiers composés (C_1) ;
- Une étape (E6) d'ajout d'un deuxième tampon de précipitation (TP_2) audit filtrat (F_1) en vue d'obtenir un deuxième précipité (P_2) contenant des molécules d'ADN cellulaire de l'espèce cible ;
- Une étape (E7) de filtration dudit filtrat contenant ledit deuxième précipité (P_2) contre un deuxième filtre (FT_2), en vue de séparer des deuxièmes composés (C_2) issus dudit deuxième précipité (P_2), ledit deuxième filtre (FT_2) étant choisi pour ne pas laisser passer lesdites molécules d'ADN cellulaire précipitées de l'espèce cible ;
- Une étape (E9) d'élution des molécules d'ADN cellulaire de l'espèce cible présente dans les deuxièmes composés issus du deuxième précipité (P_2) à travers le deuxième filtre (FT_2), par ajout d'un tampon d'élution (TE_1) choisi pour dissoudre lesdites molécules d'ADN cellulaire, et de récupération d'un éluat (E_1) ;
- Une étape de détection des molécules d'ADN cellulaire de l'espèce cible présentes dans ledit éluat (E_1) ;

2. Procédé selon la revendication 1, **caractérisé en ce qu'**il comporte également une étape (E8) de lavage des deuxièmes composés (C_2) issus du deuxième précipité (P_2) par passage d'un tampon de lavage (TL_1) à travers le deuxième filtre (FT_2).

3. Procédé selon la revendication 2, **caractérisé en ce que** le tampon de lavage (TL_1) est à base d'alcool ou d'alcool et d'eau, ou d'alcool, eau et sels.

4. Procédé selon l'une des revendications 1 à 3, **caractérisé en ce qu'**il comporte une étape (E8) de séchage des deuxièmes composés (C_2) obtenus après filtration sur le deuxième filtre (FT_2).

5. Procédé selon l'une des revendications 1 à 4, **caractérisé en ce que** la solution corrosive (S_corr) comporte un ou plusieurs des composants de la liste suivante :
- Urée,
- Sels de guanidine,
- Perchlorate ou hypochlorite de sodium,
- Potasse,
- Soude,
- Acide sulfurique, acide chlorhydrique, acide nitrique,
- Eau Oxygénée,
- Détergent ou Agent tensioactif (Tween, Triton, SDS, Tergitol),
- Chlorure de Sodium ou de Potassium.

6. Procédé selon l'une des revendications 1 à 5, **caractérisé en ce que** le premier tampon de précipitation (TP_1) comporte un ou plusieurs des composants de la liste suivante :
- Acétate d'ammonium,
- Polyvinylpyrrolidone.

7. Procédé selon l'une des revendications 1 à 6, **caractérisé en ce que** le deuxième tampon de précipitation (TP_2) est un alcool.

8. Procédé selon la revendication 7, **caractérisé en ce que** l'alcool utilisé comme deuxième tampon de précipitation (TP_2) est l'éthanol ou l'isopropanol.

9. Procédé selon l'une des revendications 1 à 8, **caractérisé en ce que** le tampon d'élution (TE_1) est choisi parmi l'eau et au moins un réactif utilisé pour la mise en oeuvre d'une réaction d'amplification.

10. Procédé selon l'une des revendications 1 à 9, **caractérisé en ce que** l'éluat (E_1) est injecté dans plusieurs chambres fluidiques en parallèle.

11. Procédé selon l'une des revendications 1 à 10, **caractérisé en ce que** l'étape (E6) d'ajout du deuxième tampon de précipitation (TP_2) et les étapes suivantes sont mises en oeuvre dans une carte microfluidique (CM).
